# EUROPEAN PATENT APPLICATION

(11) **EP 1 371 704 A2**
(43) Date of publication of application: **17.12.2003**
(21) Application number: 03253770.6
(22) Date of filing: 13.06.2003
(51) Int. Cl.: C09J 7/02, C09J 151/00, C08F 265/04

(54) **Adhesive compositions**

(30) Priority: 14.06.2002 US 389043 P; 30.09.2002 US 414598 P
(71) Applicant: ROHM AND HAAS COMPANY, Philadelphia, Pennsylvania 19106-2399 (US)
(72) Inventor: Lester, Christopher L., Telford, Pennsylvania 18969 (US); Lofton, Elizabeth P., Ambler, Pennsylvania 19002 (US); Lorah, Dennis P., Lansdale, Pennsylvania 19446 (US)
(74) Representative: Kent, Venetia Katherine

(57) **Abstract**

Adhesive compositions are provided that impart to the adhesive articles made therefrom at least one of the following useful properties: in the wet composition, good rheology and wet film integrity (*i.e*., green strength); in the dried composition, good specific adhesion, peel-build characteristics, resistance to plasticizer and solvent migration, gas-barrier properties, improved rheology for low-temperature processing, and reduced edge ooze and flow to impart improved converting, cutting, and stripping to the adhesive article. Also provided are methods of making such adhesive articles and the adhesive articles made thereby.

## Description

### BACKGROUND:

This invention relates to a method of preparing an adhesive article and to an adhesive article formed thereby. Adhesive articles are prepared by bonding one or more substrates with an adhesive composition. In some cases, two or more substrates are both bonded to the same layer of an adhesive composition, forming a bonded composite article.

Some adhesive articles are made of relatively thin, flat layers. When the adhesive composition of such thin articles are suitable as pressure sensitive adhesives (PSAs), such adhesive articles are generally known as tapes or labels, adhesive tapes or labels, or pressure sensitive tapes or labels. Regardless of the type of adhesive composition, when two relatively thin, flat substrates are bonded with a thin layer of adhesive composition, the result is often called a laminate. The method of this invention is useful for preparing various types of composite articles, including laminates, especially flexible laminates. Laminates are used to provide packaging which is light-weight and flexible. Typically, laminates are formed from combinations of various polymeric films bonded by a bonding composition to identical polymeric films, to different films, and/or to metal foils. It is desirable to use the bonding composition at a low application weight to minimize the weight of the laminate, to maintain flexibility, and to minimize cost. The method of this invention is also useful for preparing adhesive tapes and/or labels.

New coating and bonding methods are desired which allow the preparation of adhesive or composite articles, including those made from opaque substrates. One approach to these goals has been to apply a layer of adhesive in the liquid state and then expose that layer of liquid to elevated temperature and/or to radiation such as, for example, ultraviolet (UV) radiation or electron beam (E-beam) radiation.

Adhesives cured by radiation have been disclosed by US Patent Application 2002/0016381, which describes adhesives containing vinyl modified block copolymers. However, vinyl modified block copolymers are uncommon materials, which are potentially expensive and potentially difficult to make and/or obtain. Furthermore, formulations containing vinyl modified block copolymers sometimes have very high viscosities, making the formulation difficult to coat onto a substrate. A class of ingredients that is useful in making adhesives is the class of polymeric nanoparticles (PNPs); some methods of making and using PNPs are disclosed in US Patent Application 10/097256.

An object of the present invention is to provide adhesive compositions having at least one of the following properties that are improved relative to that of the adhesive absent the PNPs^{:} in the wet composition, improved rheology and wet film integrity *(i.e.,* green strength); in the dried composition, improved specific adhesion, peel-build characteristics, resistance to plasticizer and solvent migration, gas-barrier properties, improved rheology for low-temperature processing, and reduced edge ooze and flow to impart improved converting, cutting, and stripping to the adhesive article.

### STATEMENT OF THE INVENTION:

In a first aspect of the present invention, there is provided an adhesive article comprising at least one substrate and at least one adhesive composition, wherein said adhesive composition comprises 80 weight% to 100 weight%, based on the total weight of all polymeric ingredients, polymeric nanoparticles having a mean particle diameter of 1 nm to 50 nm, wherein said polymeric nanoparticles comprise, as polymerized units, at least one multiethylenically unsaturated monomer.

In a second aspect of the present invention, there is provided an adhesive article comprising at least one substrate and at least one adhesive composition, wherein said adhesive composition is made by a process comprising:
(a) providing a dispersion of polymeric nanoparticles in a nonaqueous medium, wherein said polymeric nanoparticles have a mean particle diameter of 1 nm to 50 nm, and wherein said polymeric nanoparticles comprise, as polymerized units, at least one multiethylenically unsaturated monomer, and
(b) polymerizing at least one polymer in said nonaqueous medium.

In a third aspect of the present invention, there is provided an adhesive article comprising at least one substrate and at least one adhesive composition, wherein said adhesive composition is made by a process comprising:
(a) providing a dispersion of polymeric nanoparticles in a nonaqueous medium, wherein said polymeric nanoparticles have a mean particle diameter of 1 nm to 50 nm, and wherein said polymeric nanoparticles comprise, as polymerized units, at least one multiethylenically unsaturated monomer, and
(b) admixing said dispersion with at least one polymer.

In a fourth aspect of the present invention, there is provided an adhesive article comprising at least one substrate and at least one adhesive composition, wherein said adhesive composition is made by a process comprising:
(a) providing a concentrate of polymeric nanoparticles, wherein said concentrate has 80 weight% to 100 weight% polymeric nanoparticles based on the total weight of said concentrate, wherein said polymeric nanoparticles have a mean particle diameter of 1 nm to 50 nm, and wherein said polymeric nanoparticles comprise, as polymerized units, at least one multiethylenically unsaturated monomer, and
(b) admixing said dispersion with at least one polymer.

In a fifth aspect of the present invention, there is provided an adhesive article comprising at least one substrate and at least one adhesive composition, wherein said adhesive composition is made by a process comprising:
(a) providing a dispersion of droplets in an aqueous medium, wherein said droplets comprise monomer molecules and polymeric nanoparticles, wherein said polymeric nanoparticles have a mean particle diameter of 1 nm to 50 nm, and wherein said polymeric nanoparticles comprise, as polymerized units, at least one multiethylenically unsaturated monomer, and
(b) polymerizing said monomer molecules by a method selected from the group consisting of suspension polymerization, mini-emulsion polymerization, micro-emulsion polymerization, other in-situ polymerization methods, and mixtures thereof.

In a sixth aspect of the present invention, there is provided a method for forming an adhesive article comprising applying a layer of at least one adhesive composition to a substrate, wherein said adhesive composition comprises 80 weight% to 100 weight%, based on the total weight of all polymeric ingredients, polymeric nanoparticles having a mean particle diameter of 1 nm to 50 nm, wherein said polymeric nanoparticles comprise, as polymerized units, at least one multiethylenically unsaturated monomer.

In a seventh aspect of the present invention, there is provided a method for forming an adhesive article comprising applying a layer of at least one adhesive composition to a substrate, wherein said adhesive composition is made by a process comprising:
(a) providing a dispersion of polymeric nanoparticles in a nonaqueous medium, wherein said polymeric nanoparticles have a mean particle diameter of 1 nm to 50 nm, and wherein said polymeric nanoparticles comprise, as polymerized units, at least one multiethylenically unsaturated monomer, and
(b) polymerizing at least one polymer in said nonaqueous medium.

In a eighth aspect of the present invention, there is provided a method for forming an adhesive article comprising applying a layer of at least one adhesive composition to a substrate, wherein said adhesive composition is made by a process comprising:
(a) providing a dispersion of polymeric nanop articles in a nonaqueous medium, wherein said polymeric nanoparticles have a mean particle diameter of 1 nm to 50 nm, and wherein said polymeric nanoparticles comprise, as polymerized units, at least one multiethylenically unsaturated monomer, and
(b) admixing said dispersion with at least one polymer.

In a ninth aspect of the present invention, there is provided a method for forming an adhesive article comprising applying a layer of at least one adhesive composition to a substrate, wherein said adhesive composition is made by a process comprising:
(a) providing a concentrate of polymeric nanoparticles, wherein said concentrate has 80 weight% to 100 weight% polymeric nanoparticles based on the total weight of said concentrate, wherein said polymeric nanoparticles have a mean particle diameter of 1 nm to 50 nm, and wherein said polymeric nanoparticles comprise, as polymerized units, at least one multiethylenically unsaturated monomer, and
(b) admixing said dispersion with at least one polymer.

In a tenth aspect of the present invention, there is provided a method for forming an adhesive article comprising applying a layer of at least one adhesive composition to a substrate, wherein said adhesive composition is made by a process comprising:
(a) providing a dispersion of droplets in an aqueous medium, wherein said droplets comprise monomer molecules and polymeric nanoparticles, wherein said polymeric nanoparticles have a mean particle diameter of 1 nm to 50 nm, and wherein said polymeric nanoparticles comprise, as polymerized units, at least one multiethylenically unsaturated monomer, and
(b) polymerizing said monomer molecules by a method selected from the group consisting of suspension polymerization, mini-emulsion polymerization, micro-emulsion polymerization, other in-situ polymerization methods, and mixtures thereof.

In an eleventh aspect of the present invention, there is provided an adhesive composition made by a process comprising:
(a) providing a dispersion of polymeric nanoparticles in a nonaqueous medium, wherein said polymeric nanoparticles have a mean particle diameter of 1 nm to 50 nm, and wherein said polymeric nanoparticles comprise, as polymerized units, at least one multiethylenically unsaturated monomer, and
(b) polymerizing at least one polymer in said nonaqueous medium.

In a twelfth aspect of the present invention, there is provided an adhesive composition made by a process comprising:
(a) providing a dispersion of polymeric nanoparticles in a nonaqueous medium, wherein said polymeric nanoparticles have a mean particle diameter of 1 nm to 50 nm, and wherein said polymeric nanoparticles comprise, as polymerized units, at least one multiethylenically unsaturated monomer, and
(b) admixing said dispersion with at least one polymer.

In a thirteenth aspect of the present invention, there is provided an adhesive composition made by a process comprising
(a) providing a concentrate of polymeric nanoparticles, wherein said concentrate has 80 weight% to 100 weight% polymeric nanoparticles based on the total weight of said concentrate, wherein said polymeric nanoparticles have a mean particle diameter of 1 nm to 50 nm, and wherein said polymeric nanoparticles comprise, as polymerized units, at least one multiethylenically unsaturated monomer, and
(b) admixing said concentrate with at least one polymer.

In a fourteenth aspect of the present invention, there is provided an adhesive composition made by a process comprising:
(a) providing a dispersion of droplets in an aqueous medium, wherein said droplets comprise monomer molecules and polymeric nanoparticles, wherein said polymeric nanoparticles have a mean particle diameter of 1 nm to 50 nm, and wherein said polymeric nanoparticles comprise, as polymerized units, at least one multiethylenically unsaturated monomer, and
(b) polymerizing said monomer molecules by an in-situ polymerization method.

### DETAILED DESCRIPTION

The practice of the present invention involves the use of an adhesive composition that contains polymeric nanoparticles. The adhesive composition optionally also contains one or more polymers, and the adhesive composition may optionally further contain other ingredients.

As used herein, the term "dispersion" refers to a physical state of matter that includes at least two phases wherein a first phase is distributed in a second phase, the second phase being a continuous medium. The continuous medium is also called a "dispersion medium." An "aqueous" medium is meant herein a medium that is from 50 weight% to 100 weight% water, based on the weight of the aqueous medium. A "non-aqueous" medium is meant herein a medium that is from 0 weight% to less than 50 weight% water, based on the weight of the nonaqueous medium. By "aqueous dispersion" and "nonaqueous dispersion" are meant herein, respectively, a dispersion in an aqueous medium and a dispersion in a nonaqueous medium.

The term "(meth)acrylic" used herein includes both acrylic and methacrylic and the term "(meth)acrylate" includes both acrylate and methacrylate. Likewise, the term "(meth)acrylamide" refers to both, acrylamide and methacrylamide. "Alkyl" includes straight chain, branched and cyclic alkyl groups.

By "fluid" is meant herein a liquid with viscosity of 25 Pa•s (25,000 cps) or less, as measured by standard methods, for example using Brookfield viscometer model DVI with a #25 spindle. The temperature at which the viscosity is measured is the temperature at which application to substrate is contemplated.

A "curable" moiety as used herein is a moiety that contains chemical reactivity sufficient to form, upon exposure to one or more cure conditions, chemical attachment to at least one other moiety. The curable moiety forms a chemical attachment to a moiety that is the same or different. For example, the curable moiety may form a chemical attachment to a polymeric nanoparticle, to a substrate, to other ingredients of the adhesive composition, or to the polymeric composition. Chemical attachment herein means that the curable moiety and the other moiety can be re-separated only by further chemical reaction and not by physical means such as dissolution in solvent. In many embodiments, curable moieties will be curable because they contain functional groups that are capable of reacting, upon exposure to cure conditions, with identical, similar, or complementary (as defined herein below) reactive groups.

Monomers are polymerizable compounds with relatively low molecular weight, usually 1,000 or less. Oligomers are linear, branched, or star-structured compounds of 2 to 10 monomer units; molecular weights of oligomers vary according to the molecular weight of the monomer units, but typical oligomers have molecular weights (Mn, or number-average molecular weight, as measured by gel permeation chromatography) of 10,000 or less. Polymers are linear, branched, comb-structured, star-structured, or crosslinked compounds of 11 or more monomer units, typically with Mn of more than 10,000. The term "resin" is used to mean either a polymer or an oligomer.

As used herein, "solids" or "solid ingredients" means PNPs, polymerizable compounds, polymers and remaining *(i.e.,* other than PNPs, polymers, and polymerizable compounds) solid ingredients. Remaining solid ingredients are those that, in pure form, are solid at 25°C. The solids level of a composition is the sum of the weights of all solids, expressed as a percentage of the total weight of the composition. The solids weight% of an ingredient of a composition based on total solids weight of that composition means 100 times the weight of that ingredient, isolated by itself, divided by the solids level of that composition.

In some embodiments of the present invention, the adhesive composition will be useful as a pressure sensitive adhesive (PSA), as a dry bond laminating adhesive, as a cold seal adhesive, and/or as a heat seal adhesive. Generally, PSA adhesive compositions are applied to a first substrate and dried; this combination of first substrate and dried adhesive composition is one example of an adhesive article of the present invention. The dried PSA is often stored in contact with a release surface until it is desired to form a composite article by removing the release surface and then contacting the dried PSA with a second substrate.

In some embodiments, the adhesive composition is applied as a fluid to a substrate; the fluid may contain a solvent or dispersion medium; alternatively, the fluid may have no solvent or dispersion medium. When the adhesive composition is in the fluid state, it is called herein the "wet" composition. In some embodiments the adhesive composition is wet because of the presence of a solvent or dispersion medium; in other embodiments, because the ingredients form a fluid without the presence of a solvent or dispersion medium; in other embodiments, because the adhesive composition has been heated to a temperature high enough to make the adhesive composition melt or otherwise become a fluid. In some embodiments, two or more of these causes will operate simultaneously to make the adhesive composition be wet.

In some embodiments of the present inventions, the adhesive composition changes from being a wet composition to becoming a "dry" composition, which means herein that the adhesive composition is no longer a fluid (that is, its viscosity becomes too high for the adhesive composition to function as a fluid). In some embodiments, the wet adhesive composition becomes dry because the solvent or dispersion medium evaporates; in other embodiments, because ingredients in the fluid polymerize and/or cure; in other embodiments, because the fluid cools sufficiently to have too high a viscosity to function as a fluid. In some embodiments, two or more of these causes will operate simultaneously to make the adhesive composition be dry.

The process of changing from wet to dry will be known herein as "drying." Conditions believed to improve the process of drying will be known herein as "drying conditions" (for example, among embodiments that dry by a process of solvent evaporation, some embodiments will employ high temperature and/or forced air to improve the speed and/or efficiency of drying). It is contemplated that during drying, other process may also take place, such as, for example, chemical reactions such as, for example, curing (which includes polymerization and/or crosslinking); and physical processes such as, for example, flocculation, coagulation, fusion of polymer particles, entanglement of polymer chains, and combinations thereof. It is further contemplated that, in some embodiments, drying will take place over time (for example, if drying occurs by a process that includes a relatively slow polymerization), so the drying conditions will include leaving the wet adhesive composition undisturbed (either in contact with substrates or exposed to the atmosphere) for sufficient time for drying to take place.

In embodiments that involve curing reactions in response to the drying conditions (herein called "curing embodiments"), the adhesive composition and the drying conditions will preferably be chosen so that the curing reactions proceed in a useful way. In the practice of curing embodiments, drying conditions will be synonymous with "curing conditions."

The practice of the present invention includes the use of polymeric particles having a mean diameter in the range of from 1 nanometer (nm) to 50 nm, the particles including, as polymerized units, at least one multiethylenically unsaturated monomer. The polymeric particles, referred to herein as polymeric nanoparticles ("PNPs"), are addition polymers, which contain, as polymerized units, at least one multiethylenically unsaturated monomer. Suitable multiethylenically unsaturated monomers useful in the present invention include di-, tri-, tetra-, or higher multifunctional ethylenically unsaturated monomers such as, for example, divinyl benzene, trivinylbenzene, divinyltoluene, divinylpyridine, divinylnaphthalene, divinylxylene, ethyleneglycol di(meth)acrylate, trimethylolpropane tri(meth)acrylate, diethyleneglycol divinyl ether, trivinylcyclohexane, allyl (meth)acrylate, diethyleneglycol di(meth)acrylate, propyleneglycol di(meth)acrylate, trimethylolpropane tri(meth)acrylate, 2,2-dimethylpropane-1,3-di(meth)acrylate, 1,3-butylene glycol di(meth)acrylate, 1,4-butanediol di(meth)acrylate, 1,6-hexanediol di(meth)acrylate, tripropylene glycol di(meth)acrylate, triethylene glycol di(meth)acrylate, polyethylene glycol di(meth)acrylates, such as polyethylene glycol 200 di(meth)acrylate and polyethylene glycol 600 di(meth)acrylate, tetraethylene glycol di(meth)acrylate, ethoxylated bisphenol A di(meth)acrylate, poly(butanediol) di(meth)acrylate, pentaerythritol tri(meth)acrylate, trimethylolpropane triethoxy tri(meth)acrylate, glyceryl propoxy tri(meth)acrylate, pentaerythritol tetra(meth)acrylate, dipentaerythritol monohydroxypenta(meth)acrylate, divinyl silane, trivinyl silane, dimethyl divinyl silane, divinyl methyl silane, methyl trivinyl silane, diphenyl divinyl silane, divinyl phenyl silane, trivinyl phenyl silane, divinyl methyl phenyl silane, tetravinyl silane, dimethyl vinyl disiloxane, poly(methyl vinyl siloxane), poly(vinyl hydro siloxane), poly(phenyl vinyl siloxane), and mixtures thereof.

Typically, the PNPs contain at least 1% by weight based on the weight of the PNPs, of at least one polymerized multiethylenically unsaturated monomer. Up to and including 100% polymerized multiethylenically unsaturated monomer, based on the weight of the PNPs, can be effectively used in the particles of the present invention. It is preferred that the amount of polymerized multiethylenically unsaturated monomer is from 1% to 80%, more preferably from 1% to 60%, most preferably from 1% to 25%, by weight based on the weight of the PNPs.

The PNPs optionally contain as polymerized units one or more of a variety of other monomers that are not multiethylenically unsaturated monomers. Suitable amount of monomers that are not multiethylenically unsaturated in the PNPs, as polymerized units, is 0% to 99%, based on the weight of the PNPs; preferred is 20% to 99%; more preferred is 40% to 99%; most preferred is 75% to 99%. Some suitable other monomers include, for example, C₁-C₂₄ alkyl (meth)acrylates such as methyl (meth)acrylate, ethyl (meth)acrylate, propyl (meth)acrylate, butyl (meth)acrylate, isobutyl (meth)acrylate, hexyl (meth)acrylate, cyclohexyl (meth)acrylate, 2-ethylhexyl (meth)acrylate, octyl (meth)acrylate, decyl (meth)acrylate, dodecyl (meth)acrylate, pentadecyl (meth)acrylate, hexadecyl (meth)acrylate, octadecyl (meth)acrylate, and nonadecyl (meth)acrylate, and mixtures thereof. Other suitable monomers include, for example, 2-hydroxyalkyl (meth)acrylates such as 2-hydroxyethyl (meth)acrylate, 2-hydroxypropyl (meth)acrylate, 1 -methyl-2-hydroxyethyl (meth)acrylate, and 2-hydroxybutyl (meth)acrylate. Also suitable are vinyl acetate, vinyl versatate, and diisobutylene; ureido containing monomers such as N-(ethyleneureidoethyl)-4-pentenamide, N-(ethylenethioureido-ethyl)-10-undecenamide, butyl ethyleneureido-ethyl fumarate, methyl ethyleneureido-ethyl fumarate, benzyl N-(ethyleneureido-ethyl) fumarate, and benzyl N-(ethyleneureido-ethyl) maleamate. Also suitable are vinylaromatic monomers such as styrene, α-methylstyrene, vinyltoluene, p-methylstyrene, ethylvinylbenzene, vinylnaphthalene, vinylxylenes, and nonylphenoxy propenyl polyethoxylated alcohol. The vinylaromatic monomers also include their corresponding substituted counterparts, such as halogenated derivatives, i.e., containing one or more halogen groups, such as fluorine, chlorine or bromine; and nitro, cyano, (C₁-C₁₀)alkoxy, halo(C₁-C₁₀)alkyl, (C₁-C₁₀)alkoxy, carboxy, and the like.

Also suitable for inclusion as polymerized units in some embodiments of the PNP of the present invention are substituted ethylene monomers including, for example, allylic monomers, vinyl acetate, vinyl formamide, vinyl chloride, vinyl fluoride, vinyl bromide, vinylidene chloride, vinylidene fluoride and vinylidene bromide.

In some embodiments, the PNPs further contain, as polymerized units, at least one water soluble monomer. By "water soluble monomer" herein is meant a monomer having a solubility in water of at least 7 weight %, preferably at least 9 weight %, and most preferably as least 12 weight %, at a temperature of 25 °C. Data for the water solubility of monomers is found, for example, in "Polymer Handbook" (Second Edition, J. Brandrup, E.H. Immergut, Editors, John Wiley & Sons, New York) and "Merck Index"(Eleventh Edition, Merck & Co, Inc., Rahway, New Jersey). Examples of water soluble monomers include ethylenically unsaturated ionic monomers and ethylenically unsaturated water soluble nonionic monomers. Monomers that are not water soluble are known herein as "water insoluble" monomers.

In some embodiments, the PNPs contain as polymerized units at least one ionic ethylenically unsaturated monomer, by which is meant herein that the monomer would bear an ionic charge if the PNPs were dispersed in an aqueous medium. The ionic ethylenically unsaturated monomer is referred to herein as "ionic monomer". The ionic monomer may be multiethylenically unsaturated or it may not be. Suitable ionic monomers include, for example, acid-containing monomers, base-containing monomers, quaternized nitrogen-containing monomers, and other monomers that can be subsequently formed into ionic monomers such as monomers which can be neutralized by an acid-base reaction to form an ionic monomer. Suitable acid groups include carboxylic acid groups and strong acid groups such as phosphorus containing acids and sulfur containing acids. Suitable base groups include amines and amides.

In embodiments of the present invention in which the PNP includes acid-containing monomers as polymerized units, suitable acid-containing monomers include, for example, carboxylic acid monomers, such as (meth)acrylic acid, acryloxypropionic acid, and crotonic acid; dicarboxylic acid monomers such as itaconic acid, maleic acid, fumaric acid, and citraconic acid; and monomers with half esters of dicarboxylic acid groups such as monomers containing one carboxylic acid functionality and one C₁₋₆ ester. Preferred are acrylic acid, methacrylic acid, and mixtures thereof. Also suitable are strong acid monomers, which include, for example, sulfur acid monomers such as, for example, 2-acrylamido-2-methyl propane sulfonic acid, styrene sulfonic acid, styrene sulfinic acid, and vinyl sulfinic acid; and phosphorus acid monomers such as 2-phosphoethyl (meth)acrylate, vinyl phosphoric acid, vinyl phosphinic acid. Other suitable acid monomers include terminally unsaturated acid containing macromonomers as disclosed in U.S. Patent No. 5,710,227. Phosphorus acid monomers are desirable as they can provide improved adhesion to certain substrates (e.g., metal).

In embodiments of the present invention in which the PNP includes base-containing monomers as polymerized units, suitable base-containing monomers include, for example, monomers having amine functionality, which include N,N-dimethylaminoethyl (meth)acrylate, N,N-diethylaminoethyl (meth)acrylate, N-t-butylaminoethyl (meth)acrylate, N,N-dimethylaminopropyl (meth)acrylamide, p-aminostyrene, N,N-cyclohexylallylamine, allylamine, diallylamine, dimethylallylamine, N-ethyldimethylallylamine, crotyl amines, and N-ethylmethallylamine; monomers having pyridine functionality, which includes 2-vinylpyridine and 4-vinylpyridine; monomers having piperidine functionality, such as vinylpiperidines; and monomers having imidazole functionality, which includes vinyl imidazole. Other suitable base-containing monomers include oxazolidinylethyl (meth)acrylate, vinylbenzylamines, vinylphenylamines, substituted diallylamines, 2-morpholinoethyl (meth)acrylate, methacrylamidopropyl trimethyl ammonium chloride, diallyl dimethyl ammonium chloride, 2-trimethyl ammonium ethyl methacrylic chloride, and the like.

In some embodiments, the PNPs may contain as polymerized units at least one amphoteric monomer. Suitable amphoteric monomers include, for example, N-vinylimidazolium sulfonate inner salts and N,N-Dimethyl-N-(3-methacrylamidopropyl)-N-(3-sulfopropyl) ammonium betaine.

In some embodiments, the PNPs contain as polymerized units at least one ethylenically unsaturated water soluble nonionic monomer (referred to herein as "water soluble nonionic monomer"). Examples of water soluble nonionic monomers include hydroxyalkyl (meth)acrylates such as hydroxyethyl (meth)acrylate and hydroxypropyl (meth)acrylate; poly(alkylene oxide) esters of (meth)acrylic acid such as poly(ethylene oxide)20 methacrylate and poly(propylene oxide)₁₅₀ acrylate; acrylamide; and methacrylamide.

The PNPs of the present invention optionally contain functional groups, which have been provided by including, as polymerized units, monomers containing functional groups (also called "functional monomers" herein); functional groups introduced in this way are known herein as "primary" functional groups. Other functional groups can be optionally attached to the PNPs by taking PNPs with primary functional groups and reacting those primary functional groups with suitable modifying compounds, as taught in US 5,270,380. Generally, the reaction between the modifying compound and the primary functional groups will be non-radical. A suitable modifying compound is any compound that reacts usefully with the primary functional groups of the PNPs; however, modifying compounds are thought to be most useful when they are used to alter the functionality of the PNP. That is, most modifying compounds will have at least one "linking" functional group and at least one "secondary functional" group on the same molecule. Generally, the linking functional groups will react with the primary functional groups of the PNPs to form bonds between the modifying compounds and the PNPs; in this way, some or all of the primary functional groups of the PNPs will be converted to secondary functional groups. It is contemplated that secondary functional groups could be further modified by reaction with subsequent modifying compounds.

Various functional groups are suitable for use in the present invention. Any suitable functional group may be used as primary functional group, a linking functional group, and/or as a secondary functional group. Suitable functional groups include, for example, acetoacetate, aldehyde, amine or other base, anhydride, isocyanate, epoxy, hydrazide, carboxyl or other acid, carbodiimide, halide, chloro-methyl ester, chloromethyl amine, hydroxyl, aziridine, mercaptan, unsaturation, thiol, and mixtures thereof.

In the practice of the present invention, whenever one functional group can be reacted with a different functional group to form a useful bond, such a pair of functional groups is said herein to be "complementary." For example a hydroxyl functional group on first moiety may be made to react with a carboxyl functional group on a second moiety to form a bond (in this example, an ester linkage) between the moieties. Pairs of functional groups that are complementary include, for example: (a) acetoacetate-aldehyde; (b) acetoacetate-amine; (c) amine-aldehyde; (d) amine-anhydride; (e) amine-isocyanate; (f) amine-epoxy; (g) aldehyde-hydrazide; (h) acid-epoxy; (i) acid-carbodiimide; (j) acid-chloro methyl ester; (k) acid-chloro methyl amine; (1) acid-alcohol; (m) acid-anhydride; (n) acid-aziridine; (o) epoxy-mercaptan; and (p) isocyanate-alcohol.

In embodiments of the present invention that use modifying compounds, the reaction between the primary functional groups of the PNPs and the linking functional groups of the modifying compounds alternatively provide either ionic or covalent binding. Appropriate ionic binding includes acid-base interaction and ion pair binding of negatively and positively charged atoms. Covalent binding may be provided by conducting a chemical reaction between complementary functional groups on the PNPs *(i.e.,* the "primary" functional groups) and on the modifying compounds *(i.e.,* the "linking" functional group). In any pair of complementary reactive groups, the first or second reactable group in each pair can be present in the PNPs or in the modifying compound.

In the practice of the present invention, an example of providing epoxy functionality as a primary functional group on PNPs would be PNPs made by including glycidyl (meth)acrylate and/or allyl glycidyl ether as polymerized units in the PNPs. Other monomers suitable for providing primary functionality include, for example, anhydride, such as maleic anhydride, an ester such as methyl acrylate, and halide-containing functional monomers. Suitable halide-containing functional monomers include, for example, vinylaromatic halides and halo-alkyl(meth)acrylates. Suitable vinylaromatic halides include vinylbenzyl chloride and vinylbenzyl bromide. Suitable halo-alkyl(meth)acrylates include chloromethyl (meth)acrylate. Other suitable functional monomers include allyl chloride, allyl bromide, and (meth)acrylic acid chloride.

In the practice of the present invention, an example of providing unsaturation as a secondary functional group on PNPs would be PNPs made by including a hydroxyl-functional monomer such as, for example, hydroxyethyl methacrylate, as polymerized units in the PNPs; finished PNPs could then be reacted with acrylic acid. It is contemplated that the acid groups of acrylic acid will react with the hydroxyl groups of the PNPs, resulting in PNPs with acrylic functionality, including the unreacted double bond of the acrylic group, as secondary functionality of the PNPs.

In the practice of the present invention, one suitable method for providing PNPs is that of preparing a nonaqueous PNP dispersion containing the PNPs dispersed in at least one solvent. By "nonaqueous" medium herein is meant a medium that contains from zero weight% to less than 50 weight% water, based on the weight of the nonaqueous medium.

A suitable polymerization process to prepare the nonaqueous PNP dispersion is free radical solution polymerization of at least one multiethylenically unsaturated monomer and, optionally, at least one other monomer. By "solution polymerization" herein is meant free radical addition polymerization in a suitable solvent for the polymer. By "suitable solvent for the polymer" herein is meant that linear random (co)-polymers having substantially similar polymerized monomer units to the PNPs, are soluble in the solvent. Another method for selecting a suitable solvent or mixture of solvents is on the basis of using solubility parameter analysis. According to such methods, the suitability of the solvent is determined by substantially matching the solubility parameters of the PNP and of the solvent, such as the Van Krevelen parameters of delta d, delta p, delta h and delta v. See, for example, Van Krevelen et al., Properties of Polymers. Their Estimation and Correlation with Chemical Structure, Elsevier Scientific Publishing Co., 1976; Olabisi et al., Polymer-Polymer Miscibility. Academic Press, NY, 1979; Coleman et al., Specific Interactions and the Miscibility of Polymer Blends, Technomic, 1991; and A. F. M. Barton, CRC Handbook of Solubility Parameters and Other Cohesion Parameters, 2^{nd} Ed., CRC Press, 1991. Delta d is a measure of dispersive interactions, delta p is a measure of polar interactions, delta h is a measure of hydrogen bonding interactions, and delta v is a measure of both dispersive and polar interactions. Such solubility parameters are alternatively calculated, such as by the group contribution method, or determined experimentally as is known in the art. A preferred solvent has a delta v parameter within 5 (joule per cubic centimeter)^{½}, preferably within 1 (joule per cubic centimeter)^{½} of the polymer delta v parameter. Suitable solvents for the polymerization include organic solvents such as hydrocarbons; alkanes; halohydrocarbons; chlorinated, fluorinated, and brominated hydrocarbons; aromatic hydrocarbons; ethers; ketones; esters; alcohols; and mixtures thereof. Particularly suitable solvents, depending on the composition of the PNP, include dodecane, mesitylene, xylenes, diphenyl ether, gamma-butyrolactone, ethyl acetate, ethyl lactate, propyleneglycol monomethyl ether acetate, caprolactone, 2-heptanone, methylisobutyl ketone, diisobutylketone, propyleneglycol monomethyl ether, and alkyl-alcohols, such as isopropanol, decanol, and t-butanol; and supercritical carbon dioxide.

In some embodiments, the nonaqueous PNP dispersion is prepared by first charging a solvent or, alternatively, a mixture of solvent and some portion of the monomers to a reaction vessel. The monomer charge is typically composed of monomers, an initiator, and a chain transfer agent. Typically, initiation temperatures are in the range of from 55 °C to about 125 °C, although lower or higher initiator temperatures are possible using suitable low temperature or high temperature initiators known in the art. After the heel charge has reached a temperature sufficient to initiate polymerization, the monomer charge or balance of the monomer charge is added to the reaction vessel. The monomer charge time period is typically in the range of from 15 minutes to 4 hours, although both shorter and longer time periods are envisioned. During the monomer charge, the reaction temperature is typically kept constant, although it is possible to vary the reaction temperature. After completing the monomer mixture addition, additional initiator in solvent can be charged to the reaction and/or the reaction mixture may be held for a time.

Control of PNP particle size and distribution can be achieved by one or more of such methods as choice of solvent, choice of initiator, total solids level, initiator level, type and amount of multi-functional monomer, type and amount of ionic monomer, type and amount of chain transfer agent, and reaction conditions.

Initiators useful in the free radical polymerization of the present invention include, for example, one or more of: peroxyesters, alkylhydroperoxides, dialkylperoxides, azoinitiators, persulfates, redox initiators, and the like. The amount of the free radical initiator used is typically from 0.05% to 10% by weight, based on the weight of total monomer. Chain transfer reagents are optionally used to control the extent of the polymerization of the PNPs useful in the present invention. Suitable chain transfer agents include, for example: alkyl mercaptans such as dodecyl mercaptan, aromatic hydrocarbons with activated hydrogens such as toluene, and alkyl halides such as bromotrichloroethane.

The PNPs have a mean diameter in the range of from 1 nm to 50 nm, preferably in the range of from 1 nm to 40 nm, more preferably from 1 nm to 30 nm, even more preferably from 1 nm to 25 nm, even further preferably from 1 nm to 20 nm, and most preferably from 1 nm to 10 nm. It is further typical that the PNPs have a mean particle diameter of at least 1.5 nm, preferably at least 2 nm. One method of determining the particle sizes (mean particle diameter) of the PNPs is by using standard dynamic light scattering techniques, wherein the correlation functions can be converted to hydrodynamic sizes using LaPlace inversion methods, such as CONTIN.

Typically, PNPs including as polymerized units, less than 10 weight % multiethylenically unsaturated monomer, have a glass transition temperature from -90 °C to 170 °C for the composition in the absence of the polymerized multiethylenically unsaturated monomer, as determined by a modulated differential scanning calorimetry measurement. PNPs containing as polymerized units, at least 50 weight % multiethylenically unsaturated monomer are considered to have glass transition temperatures of at least 50°C.

The PNPs of the present invention typically have an "apparent weight average molecular weight" in the range of 5,000 to 1,000,000, preferably in the range of 10,000 to 500,000 and more preferably in the range of 15,000 to 100,000. As used herein, "apparent weight average molecular weight" reflects the size of the PNP particles using standard gel permeation chromatography methods, *e.g.,* using THF solvent at 40°C, 3 Plgel™ Columns (Polymer Labs, Amherst, MA), 100 Angstrom (10 nm), 10³ Angstroms (100 nm), 10⁴ Angstroms (1 micron), 30 cm long, 7.8 mm ID, 1 milliliter per minute, 100 microliter injection volume, calibrated to narrow polystyrene standards using Polymer Labs CALIBRE™ software.

Some embodiments of the present invention involve dissolving and/or dispersing PNPs in a nonaqueous medium, so the PNPs in such embodiments must be dispersible and/or soluble in the nonaqueous medium used in the particular embodiment. In such embodiments, it is preferred to use PNPs that do not contain as polymerized units monomers with ions that are persistent in the nonaqueous medium. Monomers with "persistent" ions, as used herein, are those monomers that, after inclusion as polymerized units in the PNPs and after dissolving and/or dispersion in a nonaqueous medium, has a chemical group with an ionic charge in that medium. For example, acrylic acid is an "ionic" monomer as defined herein, but when dissolved in many organic solvents is remains in neutral form. Consequently, acrylic acid does not have a persistent ion in such solvents and is suitable for use in the present invention as polymerized units in PNPs used in such solvents. In contrast, some monomers have groups with persistent ions, such as for example some phosphorous containing acid groups and some sulfur containig acid groups; that is, such monomers remain in ionic form even when dissolved in certain organic solvents. For example, some phosphates and sulfates *(i.e.,* the ionic forms of the corresponding acids) are known to persist in ionic form in some organic solvents; it is believed such ionic forms are more likely to be persistent in nonaqueous media that contain polar solvents than in nonaqueous media that contain only nonpolar solvents. Such monomers with persistent ions are not preferred for use as polymerized units in PNPs that will be used in nonaqueous media where the ion will be persistent.

In the practice of the present invention, the PNPs may be curable, non-curable, or a mixture thereof. In some embodiments that use curable PNPs, the curable PNPs contain functional groups that are capable of reacting, upon exposure to cure conditions, with identical, similar, or complementary reactive groups. The functional groups attached to the PNPs may be primary or secondary functional groups.

The PNPs are desirably discrete or unagglomerated and dispersible, miscible or otherwise substantially compatible with/in the adhesive composition and in the polymeric composition.

In some embodiments of the present invention, in addition to the PNPs, the adhesive composition contains one or more compounds that is polymerizable. Suitable polymerizable compounds may be monomers, oligomers, resins, polymers, or mixtures thereof. For any oligomer, resin, or polymer to function as polymerizable compound, it must be capable of further polymerization during or after exposure to cure conditions. Each molecule of suitable polymerizable compound may have one or more reactive groups capable of participating in a polymerization reaction.

In embodiments where ingredients are dissolved or dispersed in a medium, preferred polymerizable compounds are those that are compatible with that medium *(i.e.,* capable of being dissolved and/or dispersed in the medium).

A preferred class of compounds suitable for use in the present invention as polymerizable compound are acrylic compounds, which are any compounds containing (meth)acrylic groups. Suitable acrylic compounds include, for example, (meth)acrylic acid, esters of (meth)acrylic acid, adducts of (meth)acrylic acid and/or (meth)acrylate esters with other functional compounds, and mixtures thereof. Among the esters of (meth)acrylic acid that are suitable for use as polymerizable compound are, for example, alkyl esters of (meth)acrylic acid; hydroxyl containing esters of (meth)acrylic acid such as for example hydroxyethyl (meth)acrylate; ring containing esters of (meth)acrylic acid such as for example isobornyl (meth)acrylate; esters of (meth)acrylic acid containing other groups such as for example ethylene oxide, allyl groups, glycidyl groups, and the like; and mixtures thereof.

Further additionally included in the class of acrylic compounds suitable for use as polymerizable compounds of the present invention are adducts of any of the above acrylic compounds with other functional compounds such as for example epoxy compounds, isocyanates, or phosphate compounds.

Yet another group of acrylic compounds suitable for use in the present invention as polymerizable compounds are acrylic curable oligomers: that is, curable oligomers made fully or partially from (meth)acrylic monomers. Curable oligomers have a functional group such as a residual acrylic double bond or any of the other functional groups disclosed herein above. Some suitable such curable oligomers are disclosed in US Patent Application Number 10/135258.

Compounds are suitable for use in the present invention because of their chemical structure, regardless of the method of synthesis or manufacture. Consequently, it is to be understood that, in the descriptions herein of chemical compounds, words like "esterified" and "adducts" and "ethoxylated" are used to describe chemical structures, regardless of the method of making those chemicals.

In addition to (meth)acrylate compounds, other polymerizable compounds are suitable for use in the present invention as polymerizable compound. Suitable compounds include for example ethylenically unsaturated compounds such as vinyl acetate, derivatives of vinyl acetate, substituted vinyl acetate compounds, styrene, substituted styrenes such as alpha-methyl styrene, and mixtures thereof. Also suitable are other compounds that are able to polymerize or copolymerize during or after exposure to cure conditions such as for example urethanes, epoxies, anhydrides, compounds capable of ring-opening polymerization, and mixtures thereof.

Another group of compounds suitable for use in the present invention as polymerizable compounds are polymers capable of further curing when exposed to cure conditions. One group of such polymers have one or two terminal ethylenically unsaturated groups; some water-insoluble examples of such polymers are disclosed in US Patent Application Number 09/951924.

Any mixtures of polymerizable compounds suitable for use in the present invention will also be suitable as polymerizable compound.

When the adhesive composition of the present invention includes polymerizable compounds, preferred are polymerizable oligomers, polymerizable polymers, and mixtures thereof; more preferred are polymerizable polymer.

In some embodiments of the present invention, in addition to the PNPs, the adhesive composition contains one or more compounds that is not polymerizable *(i.e.,* "non-polymerizable"). Examples of suitable additional non-polymerizable compounds include, for example, polymers; resins; diluents; solvents; tackifiers; pigments; emulsifiers; biocides; plasticizers; non-curable PNPs; waxes; coalescing agents; buffers; neutrallizers; antifoaming or defoaming agents; and additives that improve the flow of the composition, that help the composition wet the substrate, that reduce foaming, or that adjust the viscosity of the composition. Ingredients in the adhesive composition may be present in solution, in dispersion, or in a mixture thereof. Preferred are adhesive compositions that use little or no solvent. Also generally preferred are adhesive compositions with high solids level.

The adhesive composition of the present invention may additionally contain one or more ingredients that react chemically with other ingredients but that do not, by themselves, polymerize. Some of these chemicals become attached to one or more polymers in the adhesive composition and others do not. Examples of such compounds include, for example, those believed to improve the drying and/or curing process, such as, for example, initiators (including, for example, photoinitiators, thermal intiators, redox initiators, *etc.)* catalysts, accellerators, drying agents, chain transfer agents, crosslinking agents, and mixtures thereof.

A wide variety of crosslinking agents are suitable for use in the adhesive composition of the present invention. Those skilled in the art will recognize that the crosslinking agent and the functional groups on other moieties of the adhesive composition will desirably be chosen so that they interact with each other to form crosslinks in the dry adhesive composition. In some embodiments, some interaction between crosslinking agent and the other functional groups will occur in the wet adhesive composition. In preferred embodiments, the crosslinking agent and the other functional groups will not substantially interact in the wet adhesive composition; that is, most or all of the crosslinks will form after the adhesive composition has been applied to a substrate and been exposed to drying conditions. The crosslinks may be ionic, partially ionic, partially covalent, covalent, or mixtures thereof. One preferred class of crosslinkers are metal chelates; more preferred is aluminum acetylacetonate. When metal chelates are used, it is preferred to use them in combination with additional amounts of a chelating compound. In some embodiments, the preferred chelating compound is volatile; while the invention is not limited to a specific mechanism, it is believed that the chelating compound evaporates during the drying process, allowing the metal in the metal chelate to interact with groups in the dry adhesive composition. For example, when aluminum acetylacetonate is used, it is preferred to use it in a mixture with 2,4-pentanedione.

In some embodiments, the PNPs are used as a dispersion in the polymerization solvent. In some embodiments, the PNPs are isolated by, for example, vacuum evaporation, by precipitation into a non-solvent, and spray drying. When isolated, some PNPs can be subsequently redispersed in a medium appropriate for incorporation into an adhesive composition. It is contemplated that, if isolation is desired, the method will be chosen according to the nature of the PNPs and of the adhesive composition. Some PNPs with Tg above 20°C may yield freely flowing powders when isolated, which may be desirable in some embodiments, while some PNPs with Tg below 20°C will be tacky or otherwise difficult to handle when isolated.

In some embodiments, the polymeric components in the dry adhesive composition will be 80 solid weight% to 100 solid weight% PNPs, based on the total solid weight of all polymeric components in the dry adhesive composition. Preferred is 90 solid weight% to 100 solid weight%. These embodiments will be called "only-PNP" herein, and they may conventional non-polymeric adhesive adjuvants, as described herein above, in addition to the PNPs. These only-PNP embodiments include, for example, for example, formulations in which the PNPs are dissolved or dispersed in a continuous medium. Alternatively, in some only-PNP embodiments, the PNPs are used in a formulation without a solvent or other continuous medium. In only-PNP embodiments, the PNPs may be curable, non-curable, or a mixture thereof. If curable PNPs are included, the dry adhesive composition will sometimes contain the reaction products of the reactive groups on the PNPs. For use in PSAs, the Tg is preferably in the range of-70°C to -10°C, and more preferably, -70°C to -35°C. For dry bond laminating adhesives the Tg is preferably in the range from -50°C to 50°C, more preferably, -30°C to 25°C, still more preferably, -10°C to 10°C. For heat seal adhesives, the Tg is preferably in the range from -20°C to 50°C, more preferably, 10°C to 30°C. Additionally, the modulus for heat seal adhesives is preferably from 1x10⁶ dyne/cm to 9x10⁶ dyne/cm, more preferably 2x10⁶ dyne/cm to 5x10⁶ dyne/cm at temperatures in the range from 25°C to 100°C. For cold seal adhesives, the Tg is preferably in the range from -100°C to 10°C, and more preferably -50°C to 0°C.

In some embodiments of the present invention the wet adhesive composition includes PNPs and at least one other polymer. The other polymer may be introduced into the composition by any of a wide variety of methods, including, for example, the following: in a nonaqueous dispersion of PNPs, introducing monomer molecules into the dispersion and then polymerizing them; admixing at least one polymer with a nonaqueous dispersion of PNPs; admixing a polymer with pure or concentrated PNPs; or mixtures of these methods. Suitable polymers are any polymers known in the art, including, for example, acrylic polymers; natural rubber; synthetic rubber, including polymers and copolymers of butadiene, and isoprene; polyesters; polyurethanes; and mixtures thereof. Preferred are acrylic polymers, polyesters, polyurethanes, and mixtures and copolymers thereof; more preferred are acrylic polymers.

In embodiments of the present invention that contain PNPs and at least one other polymer, suitable solids weight percentages of the PNPs in the adhesive composition of the present invention, based on total solids weight of the adhesive composition, are typically from 0.1 solids weight% to 99 solids weight%, more typically from 0.25 solids weight% to 75 solids weight%, even more typically from 0.5 solids weight% to 50 solids weight%, further more typically from 0.75 solids weight% to 25 solids weight%, and most typically from 1 solids weight% to 10 solids weight%. When used at levels below 15 solids weight%, PNPs are referred to as additives; when used at or above 15 solids weight%, PNPs are referred to as blend agents.

In some embodiments of the present invention, the wet adhesive composition is made by a process that includes providing a dispersion of PNPs in a nonaqueous medium and then polymerizing at least one polymer in that medium. These embodiments will be called herein "second polymerization" embodiments, and polymers formed in the medium of the PNPs will be called "second polymers." It is believed that second polymerization embodiments will provide an adhesive composition in which the PNPs are homogeneously randomly distributed throughout the composition. In some second polymerization embodiments, the PNPs will have at least one functional group that reacts chemically with a second polymer; this interaction may take place during or after the polymerization. In other second-polymerization embodiments, the PNPs will not react chemically with the second polymer, though the PNPs desirably will remain dispersed in the medium after the polymerization and will desirably be dispersed in the dry adhesive composition; that is, in the dry adhesive composition, the second polymer will desirably be the continuous medium.

In some embodiments (herein called "polymer admixture" embodiments) of the present invention, a nonaqueous dispersion of PNPs is admixed with at least one other polymer. In some polymer admixture embodiments, this result will be accomplished by admixing a solution or dispersion of the other polymer with the nonaqueous dispersion of PNPs; the ingredients and mixing methods will desirably be chosen so that, in the admixture, both the PNPs and the other polymer will be dissolved and/or dispersed.

In some embodiments (called "concentrate embodiments" herein) of the present invention, pure or concentrated PNPs are admixed with a polymer. By concentrated is meant a composition that contains 80 weight% to 100 weight% PNPs based on the total weight of the composition; such a composition is called herein a PNP concentrate. PNP concentrates result from isolating PNPs, as discussed herein above. In these concentrate embodiments, the PNP concentrate is admixed with at least one other polymer, either with or without the presence of solvent or dispersion medium. In embodiments with no solvent or dispersion medium, the PNP concentrate and the other polymer will sometimes be mixed at elevated temperature in an apparatus such as an extruder, for example. In concentrate embodiments that include solvent or dispersion medium, the ingredients will desirably be chosen so that the PNPs the other polymer will dissolve or disperse in the solvent or dispersion medium.

Some embodiments of the present invention involve polymerization of aqueous monomer emulsions in the presence of PNPs. In the polymerization art, many methods are known in which polymers are produced, using aqueous monomer emulsions as one of the starting materials. While the present invention may be practiced with any of these methods, the preferred methods (herein called "in-situ polymerization" methods) involve preservation of the emulsion droplets throughout the polymerization. That is, one starting ingredient of an in-situ polymerization is an aqueous monomer emulsion, in which monomer droplets are dispersed in an aqueous medium, usually stabilized with a small amount of surfactant; then, during in-situ polymerization, most of the monomer molecules in a given droplet remain in that droplet while they undergo polymerization; thus the monomer droplet becomes a particle made mostly or completely of polymer. Some process suitable as in-situ polymerizations include, for example, suspension polymerization, mini-emulsion polymerization, micro-emulsion polymerization, and processes that are mixtures thereof.

In the some embodiments of the present invention that involve in-situ polymerization, some or all of the monomer emulsion droplets contain PNPs. In some of these embodiments, the some or all of the PNPs may be concentrated and then mixed with aqueous medium, monomer, and any other ingredients to form the PNP-containing monomer emulsion. In other embodiments, monomer and optional surfactant may be added to a nonaqueous dispersion of PNPs to form the PNP-containing monomer emulsion. However the PNP-containing monomer emulsion is formed, the in-situ polymerization is then carried out. While the present invention is not limited to any particular mechanism, it is believed that many or all of the polymer particles formed by the in-situ polymerization will contain PNPs.

In all embodiments of the present invention, the adhesive composition optionally contains other ingredients in addition to the PNPs and the optional other polymer.

In the practice of the present invention, the adhesive composition is applied onto a substrate. Application may be performed by any means, including for example, manual or mechanical spreading. Suitable application methods include for example roller coating, reverse roller coating, rod coating, gravure coating, Meyer rod coating, curtain coating, slot die coating, and the like. The adhesive composition may be applied at room temperature (20°C to 25°C); it may be applied hot *(i.e.,* at a temperature above room temperature); or it may be applied cool *(i.e.,* at a temperature below room temperature). Typically, hot application is performed if it is desired to reduce the viscosity of the adhesive composition to improve the operation of the application method. If hot application is used, the temperature is chosen to prevent or minimize polymerization during application of the adhesive composition; if hot application is performed, it is generally performed at 70°C or lower. Typically, cool application is performed if it is desired to reduce the rate of the curing reaction; cool application is often used in embodiments involving 2-pack systems (discussed herein below).

In the practice of the present invention, the layer of applied adhesive composition may form a continuous or discontinuous layer. The thickness of the applied layer of adhesive composition may be uniform or it may vary. The amount of adhesive composition that is applied to the substrate will depend on the substrates and on the use to which the composite article will be put. In some embodiments, a preferable thickness of the applied adhesive coating is from 0.5 µm to 200 µm. In some embodiments, a preferable amount of applied adhesive composition is at least 0.16 g/m² (0.1 lb/ream). Also, in some embodiments, a preferable amount of applied adhesive composition is 40 g/m² (25 lb/ream) or less. For PSAs, a preferable amount of applied adhesive composition is 15 g/m² (10 lb/ream) to 23 g/m² (15 lb/ream). For laminating adhesives, a preferable amount of applied adhesive composition is 0.8 g/m² (0.5 lb/ream) to 5 g/m² (3 Ib/ream).

In some embodiments of the present invention, after the adhesive composition is applied to a first substrate, it is contacted with a subsequent substrate to form an assembly, which is optionally subjected to applied pressure such as by passing it between rollers to effect increased contact of the substrates with the adhesive composition. In another embodiment the adhesive composition may be simultaneously or sequentially applied to two surfaces of a first substrate, which coated surfaces are then simultaneously or sequentially bonded to two subsequent substrates, which may be the same or different relative to the first substrate and each other. It is further contemplated that the composite article may subsequently be bonded to one or more other substrates using the same or a different adhesive before or after the process described herein. Also, it is contemplated that a wide variety of arrangements of substrates and polymeric adhesive layers may be used to form the composite article. For example, multiple substrates may be alternated with multiple layers of adhesive, such as for example in multilayered laminates. For another example, in some embodiments, layers of adhesive composition, each applied to its own substrate, may be brought together.

The adhesive compositions of the present invention can be applied to a substrate such as, for example, plastic, including sheets and films of substituted ethylene polymers, polypropylene, polyethylene, polyamide, polyamide imide, polyester, or composite blends; metallized films; woven and nonwoven textiles; and paper. The compositions may be applied with or without a prior substrate treatment such as a chemical primer, treated release liners, or corona or flame treated surfaces. The chemical primer can be a pre-coat of the adhesive composition with or without PNPs, or may be an alternate primer composition. The adhesive coating on the substrate is typically dried, or allowed to dry, at temperatures from 40°C to 200°C.

In some embodiments of the present invention, after the layer of adhesive composition is applied to a first substrate, some or all of that layer of adhesive composition kept out of contact with any subsequent substrate for a significant length of time, during which the adhesive composition may or may not be in contact with a release surface. After the adhesive composition is exposed to drying conditions, the combination of substrate and polymeric composition is an adhesive article. The substrate and the ingredients of the adhesive composition are desirably chosen so that the dry adhesive composition will adhere well to the substrate. These embodiments are especially useful when the adhesive composition is a PSA.

In some of these embodiments where the adhesive composition is dried before contact with any subsequent substrate, the solids level is sometimes desirably lower than the solids levels found in other embodiments. That is, as in other embodiments, the ingredients may be dissolved or dispersed in a solvent or a dispersing medium. However, it is contemplated that in the absence of subsequent substrates, the solvent and/or dispersing medium will more easily evaporate in a reasonable time during the time or soon after the time that the cure reactions take place. Thus, for low-solids coatings embodiments, suitable solids levels of the adhesive composition are 10% or higher, based on the weight of the adhesive composition; preferred are 20% or higher; more preferred is 40% or higher; still more preferred is 60% or higher.

In some embodiments (called herein "drying" embodiments), the adhesive composition becomes dry without the occurrence of any significant extent of curing. Drying embodiments involve, for example, cooling of the wet adhesive composition from the melt, evaporation of a solvent and/or dispersion medium, and/or mixtures thereof.

In some embodiments (called herein "curing" embodiments), the drying process includes significant extent of curing of curable and/or polymerizable ingredients. While the present invention is not limited to any particular mechanism or chemical reaction, it is contemplated that, in curing embodiments, upon exposure to drying conditions, any polymerizable compounds in the adhesive composition will polymerize. The polymerizable compounds may be monofunctional, multifunctional, or mixtures thereof, so that the polymers formed may be linear, branched, comb-structured, star-structured, and/or crosslinked. All such types of polymers are contemplated for use in the practice of the present invention. As used herein, "polymerization" refers to the chemical reaction of molecules to form polymers; included are reactions of curable polymers with each other or with other molecules to form larger polymers; when multifunctional molecules are included, the polymerization process may also be referred to as "crosslinking." Polymerization, with or without crosslinking, is often referred to as "curing." In the practice of the present invention, it is contemplated that the adhesive composition, upon exposure to cure condition, may undergo any or all of polymerization, curing, and/or crosslinking.

In the practice of curing embodiments of the present invention, upon exposure to cure conditions, the curable moieties *(i.e.,* polymerizable compounds and/or curable PNPs) in the adhesive composition undergo chemical reactions with each other and/or with other ingredients of the adhesive composition to cure.

In some curing embodiments of the present invention, these chemical reactions that take place are between and/or among two or more complementary reactive groups. In some curing embodiments, some moieties in the adhesive composition would have A functional groups and other moieties would have B functional groups, where A and B are complementary; such embodiments are known herein as heterogeneous-cure embodiments. The A groups and the B groups, independently, could be attached to some or all of the PNPs and to some or all of any polymerizable compounds in the adhesive composition, as long as enough A and B groups are provided so that a polymeric composition is formed upon exposure of the adhesive composition to cure condition. As examples, some embodiments include: A-functional PNPs with B-functional polymerizable compounds; A-functional PNPs with B-functional PNPs and polymerizable compounds having A and/or B functional groups. Any of the pairs of complementary functional groups discussed herein above are suitable as the A/B pairs for use in heterogeneous cure embodiments of the present invention. Either member of any complementary pair may be selected to function as either the A functional group or the B functional group, while the other member of the complementary pair will be selected to function as the B functional group or the A functional group, respectively.

It is contemplated that some curing embodiments will require, for the curing to take place, that the adhesive composition be exposed to atmospheric ingredients such as air and/or water. In practicing such embodiments, it is contemplated that exposure to such atmospheric ingredients will be accomplished as needed, for example by direct application (for example, by spraying) or by indirect application (for example by diffusion through porous substrate). In such embodiments, the adhesive composition will usually be stored away from contact with the atmosphere, to inhibit the curing reaction, until the layer or adhesive composition is applied to substrate.

In the practice of the present invention, the adhesive composition may be a one-pack system or a multi-pack system. A one-pack system is an adhesive composition in which the ingredients are all admixed together and then stored for long times, while the adhesive composition remains useful in the practice of the present invention. That is, the one-pack system may be stored, without losing either its capability of being applied as a layer or its capability of functioning as the adhesive layer in an adhesive article, for 1 week or more; preferably for 1 month or more; more preferably for 1 year or more; most preferably for 2 years or more. Embodiments of the present invention that use a one-pack system for the adhesive composition are contemplated to include drying embodiments and curing embodiments. When curing embodiments using a one-pack system are practiced, the reactive groups will be chosen to be such that the cure reaction does not take place until the adhesive composition is exposed to some cure condition other than merely making the admixture.

A multi-pack system is a combination of two or more precursor adhesive compositions that may be stored separately and then admixed with each other to form the adhesive composition of the present invention a short time before the assembly is formed. While almost any embodiment of the present invention may be practiced using a 2-pack system, it is contemplated that the use of a 2-pack system will be most advantageous when heterogeneous-cure embodiments are practiced with reactive groups that react relatively quickly with each other. For example, one pack may contain one or more moieties with functionality A (as described herein above) while another pack may contain one or more moieties with complementary functionality B; if the reaction that takes place when the packs are admixed contributes in a useful way to the formation of the polymeric composition, then this admixture of moieties with reactive groups constitutes a cure condition of the present invention.

In the practice of 2-pack embodiments of the present invention, in many cases, as the groups react with each other, the viscosity of the adhesive composition will rise. The reaction between groups should be long enough that the assembly can be assembled while the viscosity of the adhesive composition is still below 25 Pa•s (25,000 cps); preferably, the viscosity will remain below 25 Pa•s (25,000 cps) for 10 s or longer; more preferably for 1 minute or longer; most preferably for 5 minutes or longer. Generally, when the reaction that takes place upon admixture of the packs contributes in a useful way to the formation of the polymeric composition, this contribution can be observed by measuring the rise in viscosity that takes place when the packs are admixed. Preferably, the admixture reaches viscosity of 50 Pa•s (50,000 cps) in 5 days or less; more preferably in 2 days or less; even more preferably in 1 day or less.

In some 2-pack embodiments, the admixing of the moieties with reactive groups will be a sufficient cure condition to form a useful polymeric composition. In other embodiments, additional cure conditions of elevated temperature and/or radiation will be used. For embodiments using 2-pack systems, preferred cure condition is a combination of mixing reactive groups and radiation; more preferred is a combination of mixing reactive groups and electron beam radiation.

In some embodiments of the present invention, the adhesive composition is subjected to one or more curing conditions. In some embodiments, these conditions cause some or all of the adhesive composition to cure *(i.e.,* to react chemically to become a polymer). Suitable minimum extent of cure is an extent sufficient to create a bond strength that is useful and to reduce the amount of non-polymerized material to acceptable levels.

When elevated temperature is used as all or part of the drying condition of the present invention, suitable temperatures are 70°C or higher; preferred is 80°C or higher. Also preferred are temperatures low enough to avoid any substantial degradation of the adhesive article; preferred is 250°C or lower; more preferred is 200°C or lower; even more preferred is 150°C or lower. The elevated temperature is maintained for duration long enough to achieve the desired properties of the composite article; duration of elevated temperature generally will be shorter if higher temperatures are used.

For embodiments in which mixing of moieties with complementary reactive groups is used as all or part of the drying condition of the present invention, it is contemplated that the 2-pack systems would be commonly used. In such embodiments, the ingredients would be chosen so that the reaction of the complementary reactive groups was slow enough to allow the adhesive composition to be applied to a substrate before its viscosity became too high. In some embodiments, the application of the adhesive composition to a substrate would be performed at low temperature, which, it is believed, would slow the chemical reaction. In embodiments in which mixing of moieties with complementary reactive groups is the primary or only cure condition, the ingredients would be chosen so that the reaction of the complementary reactive groups was also fast enough to allow the composite article had a useful adhesive strength within a desirably short time. A useful embodiment would combine mixing of complementary reactive groups with other types of cure condition, such as elevated temperature (including possibly coating at low temperature and then curing at high temperature) and/or radiation. It is contemplated that when mixing of moieties with complementary reactive groups is used as a cure condition, the materials may become warmer than room temperature due to hot application of the adhesive composition, to exothermic chemical reactions during cure, to conversion of radiation to heat, and/or to other causes.

In some embodiments of the present invention, the adhesive composition is a one-pack system. This adhesive composition is applied to a substrate; the adhesive composition is then optionally contacted with a second substrate; and the fluid is then exposed to drying conditions.

In some embodiments of the present invention, the adhesive composition is a two-pack system. The ingredients of each pack are chosen so that when the packs are mixed together, the adhesive composition thus formed remains a fluid for more than 30 seconds but less than 24 hours. While the adhesive composition remains a fluid, it is applied to a substrate. Subsequent substrates are optionally contacted with the adhesive composition, and the fluid is then exposed to drying conditions.

In some embodiments of the present invention, the adhesive composition contains curable PNPs but no other reactive, curable, or polymerizable ingredients.

In some embodiments of the present invention, the adhesive composition contains non-curable PNPs and other ingredients that are not PNPs but that include polymerizable compounds.

In some embodiments of the present invention, the adhesive composition contains curable PNPs, other ingredients that are not PNPs but that include polymerizable compounds.

It is to be understood that for purposes of the present specification and claims that the range and ratio limits recited herein can be combined. For example, if ranges of 60 to 120 and 80 to 110 are recited for a particular parameter, it is understood that the ranges of 60 to 110 and 80 to 120 are also contemplated. Additionally, if minimum range values of 1 and 2 are recited, and if maximum range values of 3, 4, and 5 are recited, then the following ranges are all contemplated: 1 to 3, 1 to 4, 1 to 5, 2 to 3, 2 to 4, and 2 to 5.

In the following Examples, these abbreviations are used:
- MIBK =: methyl isobutyl ketone
- AA =: acrylic acid
- MMA =: methyl methacrylate
- TMPTA =: trimethylolpropane triacrylate
- BA =: n-butyl acrylate
- DEGDMA =: dietylene glycol dimethacrylate
- DMAPMA =: dimethylaminopropyl methacrylate
- TMPTA =: trimethylol propane triacrylate
- OPP =: oriented polypropylene
- mil =: 25.4 µm (0.001 inch)
- LPO =: lauryl peroxide
- SLS =: sodium lauryl sulfate
- 2-EHA =: 2-ethylhexyl acrylate
- KPS =: potassium persulfate
- MA =: methyl acrylate
- DI =: deionized

### EXAMPLES

### Example 1: Preparation of a PNPs

PNPs of MMA/BA/AA/TMPTA (35/35/20/10 wt.%) were prepared via solution polymerization as follows: A 5 liter reactor was fitted with a thermocouple, a temperature controller, a purge gas inlet, a water-cooled reflux condenser with purge gas outlet, a stirrer, and a monomer feed line. To a separate vessel was charged 450.0 g of a monomer mixture (A) consisting of 157.5 g MMA, 157.5 g BA, 90.0 g AA, and 45.00 g TMPTA. To an additional vessel was charged an initiator mix (B) consisting of 18.00 g of a 75% solution of t-amyl peroxypivalate in mineral spirits (Trigonox™ 125-C75, AkzoNobel, Chicago IL), and 112.50 g isopropyl alcohol. A charge of 2325.00 g isopropyl alcohol was added to the reactor. After sweeping the reactor with nitrogen for approximately 30 minutes, heat was applied to bring the reactor charge to 79°C. When the contents of the reactor reached 79°C, a dual feed of both the monomer mixture (A) and the initiator mix (B) were added to the reactor. The two mixtures were feed uniformly using feed pumps over 120 minutes. At the end of the monomer and initiator feeds, the batch was held at 79°C for 30 minutes before adding the first of three additional initiator charges consisting of 9.00 g of a 75% solution of t-amyl peroxypivalate in. mineral spirits (Trigonox™ 125-C75), and 22.50 g isopropyl alcohol. A second initiator charge addition was made 30 minutes after the first initiator charge addition. Similarly, the final initiator charge addition was made 30 minutes after the second initiator charge addition. The batch was then held at the polymerization temperature of 79°C for and additional 2½ hours to achieve full conversion of monomer. At the end of the final hold, the batch was neutralized with a mixture of 42.5 gm of an aqueous 28% solution of aqueous ammonia and 450.00 g water. The neutralized polymer solution was transferred to a roto-evaporator and stripped of solvent at ∼35°C at reduced pressure. The mean particle diameter was 5 nm.

### Example 2: Preparation of PNPs

Following the procedure of Example 1, a PNP of BA/AA/TMPTA (70/20/10) is prepared. The mean particle size is 5 nm.

### Example 3: Preparation of Additional PNPs

Following the procedure of Example 1, a PNP of 2-EHA/AA/TMPTA (70/20/10) is prepared. The mean particle size is 5 nm.

### Example 4: Preparation of Mini-Emulsion of Polymer/PNP Composite.

PNPs of composition 90 MMA / 10 DEGDMA are prepared in MIBK at a solids level of 5%. A 1000g sample of this composition is placed in a 2 liter flask and the MIBK removed under reduced pressure utilizing a rotary evaporator.

| Component | Amount (g) |
|---|---|
| BA | 20.9 |
| MMA | 13.9 |
| PNPs | 1.05 |
| DI Water | 144.4 |
| SLS | 0.62 |
| Hexadecane | 1.5 |
| KPS | 0.0705 |
| Sodium Bicarbonate | 0.021 |

The above mixture is placed in a reactor and homogenized for a period of 30 minutes in order to obtain a stable mini-emulsion. Then, the temperature is raised to 75°C for two hours in order to polymerize the styrene monomer. Upon cooling to room temperature, the material is filtered and characterized.

### Example 5: Preparation of mini-emulsion of polymer/PNP composite.

A mini-emulsion is prepared according to the process of Example 4 with the exception that PNPs of composition 54 MMA/26 BA / 10 DMAPMA /10 DEGDMA prepared in MIBK at a solids level of 5% is used.

### Example 6: Solventborne Adhesive Composition

A base polymer of 95 BA / 5 AA is prepared as a solution in a solvent made of 50/50 ethyl acetate/toluene and mixed with a dispersion of the PNPs of Example 2 in ethyl acetate. Mixing is performed with a benchtop mixer, stirring until the mixture is homogeneous. The resulting mixture is an adhesive composition of 60% solvent, 38% base polymer, and 2% PNPs.

The mixture is drawn down on oriented polypropylene (OPP) film of thickness 38 µm; thickness of the wet adhesive composition is 5 µm. The coated film is place in an oven at 80°C for three minutes, then closed with another piece of 38 µm OPP by applying pressure by hand with a rubber roller. The laminate is then run through a pair of rollers at a nip pressure of approximately 140 kPa at 40°C. The laminate is tested by t-peel adhesion and has approximately 250 g separation force for a sample 25.4 mm wide at 25°C and 65°C. The laminate has good adhesion at lower nip pressures and has better shear strength than similar laminates made without PNPs.

### Example 7: Solvent Adhesive with Crosslinker

A laminate is prepared as in Example 6, except that the adhesive composition further contains 0.5% aluminum acetylacetonate and 1% 2,4-pentadione.

### Example 8: Cold Seal Laminating

An adhesive composition is prepared by the method of Example 7, using the same base polymer and PNPs with composition 70 MMA / 20 AA / 10 TMPTA.

In this case two coated OPP films are prepared, with a 2.5 µm layer of wet adhesive composition, which is dried as in Example 7. The two dry adhesive layers are closed together and pressed as in Example 7. The t-peel adhesion is 350 g separation force for a sample width of 25.4 mm.

### Example 9: Second Polymerization Method

Using the methods of Example 3, a dispersion of 5 solids weight% PNPs in 50/50 ethyl acetate/tolune is prepared. A reaction vessel is purged with nitrogen, 550 g of the PNP dispersion is added and heated to reflux. 508 g 2-ethyl hexyl acrylate (2-EHA), 343 g methyl acrylate (MA), and 58 g glacial acrylic acid (AA) are mixed and prepared as a monomer solution. 45 g ethyl acetate, 74 g toluene, and 0.5 g lauryl peroxide (LPO) are mixed and prepared as an initiator feed solution. A chase solution is prepared of 61 g toluene and 5 g LPO. A dilution solution is prepared of 175 g ethyl acetate and 38 g toluene. A post-add solution is prepared of 77 g toluene, 54 g isopropyl alcohol, 2.7 g aluminum acetylacetonate, and 4.6 g 2,4-pentanedione. Then, simultaneously, the monomer solution (over 170 minutes) and the initiator solution (over 180 minutes) are added while maintaining stirring and reflux. When those feeds are finished, the nitrogen sparge is stopped and the solution is held for 1 hour. Then the chase solution is added over 120 minutes. The reaction solution is cooled to 45°C, the dilution and post-add solutions are added, and the reaction is stirred for 30 min.

The solution is coated and dried onto 50 µm (2 mil) thick polyester film, to give a dry coating thickness of 20 µm (0.8 mil). The coated film is evaluated as an adhesive tape and shows good tack, peel, shear, and converting properties; the shear and converting properties are improved over similar tapes made without PNPs.

### Example 10: Second Polymerization Method

The dispersion of PNPs of Example 3 is altered to 50 weight% solids using a rotary evaporator. The resulting dispersion is coated onto oriented polypropylene and laminated, using the methods of Example 7. The t-peel tests show desirably strong adhesion.

## Claims

1. An adhesive article comprising at least one substrate and at least one adhesive composition, wherein said adhesive composition comprises 80 weight% to 100 weight%, based on the total weight of all polymeric ingredients, polymeric nanoparticles having a mean particle diameter of 1 nm to 50 nm, wherein said polymeric nanoparticles comprise, as polymerized units, at least one multiethylenically unsaturated monomers.

2. An adhesive article comprising at least one substrate and at least one adhesive composition, wherein said adhesive composition is made by a process comprising:
(a) providing a dispersion of polymeric nanoparticles in a nonaqueous medium, wherein said polymeric nanoparticles have a mean particle diameter of 1 nm to 50 nm, and wherein said polymeric nanoparticles comprise, as polymerized units, at least one multiethylenically unsaturated monomer, and
(b) polymerizing at least one polymer in said nonaqueous medium.

3. An adhesive article comprising at least one substrate and at least one adhesive composition, wherein said adhesive composition is made by a process comprising:
(a) providing a dispersion of polymeric nanoparticles in a nonaqueous medium, wherein said polymeric nanoparticles have a mean particle diameter of 1 nm to 50 nm, and wherein said polymeric nanoparticles comprise, as polymerized units, at least one multiethylenically unsaturated monomer, and
(b) admixing said dispersion with at least one polymer.

4. An adhesive article comprising at least one substrate and at least one adhesive composition, wherein said adhesive composition is made by a process comprising:
(a) providing a concentrate of polymeric nanoparticles, wherein said concentrate has 80 weight% to 100 weight% polymeric nanoparticles based on the total weight of said concentrate, wherein said polymeric nanoparticles have a mean particle diameter of 1 nm to 50 nm, and wherein said polymeric nanoparticles comprise, as polymerized units, at least one multiethylenically unsaturated monomer, and
(b) admixing said concentrate with at least one polymer.

5. An adhesive article comprising at least one substrate and at least one adhesive composition, wherein said adhesive composition is made by a process comprising:
(a) providing a dispersion of droplets in an aqueous medium, wherein said droplets comprise monomer molecules and polymeric nanoparticles, wherein said polymeric nanoparticles have a mean particle diameter of 1 nm to 50 nm, and wherein said polymeric nanoparticles comprise, as polymerized units, at least one multiethylenically unsaturated monomer, and
(b) polymerizing said monomer molecules by an in-situ polymerization method.

6. The adhesive article of any of claims 1, 2, 3, 4, or 5, wherein said adhesive article comprises at least two substrates in contact with a layer of said adhesive composition.

7. A method for forming an adhesive article comprising applying a layer of at least one adhesive composition to a substrate, wherein said adhesive composition comprises 80 weight% to 100 weight%, based on the total weight of all polymeric ingredients, polymeric nanoparticles having a mean particle diameter of 1 nm to 50 nm, wherein said polymeric nanoparticles comprise, as polymerized units, at least one multiethylenically unsaturated monomer.

8. A method for forming an adhesive article comprising applying a layer of at least one adhesive composition to a substrate, wherein said adhesive composition is made by a process comprising:
(a) providing a dispersion of polymeric nanoparticles in a nonaqueous medium, wherein said polymeric nanoparticles have a mean particle diameter of 1 nm to 50 nm, and wherein said polymeric nanoparticles comprise, as polymerized units, at least one multiethylenically unsaturated monomer, and
(b) polymerizing at least one polymer in said nonaqueous medium.

9. A method for forming an adhesive article comprising applying a layer of at least one adhesive composition to a substrate, wherein said adhesive composition is made by a process comprising:
(a) providing a dispersion of polymeric nanoparticles in a nonaqueous medium, wherein said polymeric nanoparticles have a mean particle diameter of 1 nm to 50 nm, and wherein said polymeric nanoparticles comprise, as polymerized units, at least one multiethylenically unsaturated monomer, and
(b) admixing said dispersion with at least one polymer.

10. A method for forming an adhesive article comprising applying a layer of at least one adhesive composition to a substrate, wherein said adhesive composition is made by a process comprising
(a) providing a concentrate of polymeric nanoparticles, wherein said concentrate has 80 weight% to 100 weight% polymeric nanoparticles based on the total weight of said concentrate, wherein said polymeric nanoparticles have a mean particle diameter of 1 nm to 50 nm, and wherein said polymeric nanoparticles comprise; as polymerized units, at least one multiethylenically unsaturated monomer, and
(b) admixing said concentrate with at least one polymer.

11. A method for forming an adhesive article comprising applying a layer of at least one adhesive composition to a substrate, wherein said adhesive composition is made by a process comprising:
(a) providing a dispersion of droplets in an aqueous medium, wherein said droplets comprise monomer molecules and polymeric nanoparticles, wherein said polymeric nanoparticles have a mean particle diameter of 1 nm to 50 nm, and wherein said polymeric nanoparticles comprise, as polymerized units, at least one multiethylenically unsaturated monomer, and
(b) polymerizing said monomer molecules by an in-situ polymerization method.

12. The method of any of claims 7, 8, 9, 10, or 11, wherein said adhesive article comprises at least two substrates in contact with a layer of said adhesive composition.

13. An adhesive composition made by a process comprising:
(a) providing a dispersion of polymeric nanoparticles in a nonaqueous medium, wherein said polymeric nanoparticles have a mean particle diameter of 1 nm to 50 nm, and wherein said polymeric nanoparticles comprise, as polymerized units, at least one multiethylenically unsaturated monomer, and
(b) polymerizing at least one polymer in said nonaqueous medium.

14. An adhesive composition made by a process comprising:
(a) providing a dispersion of polymeric nanoparticles in a nonaqueous medium, wherein said polymeric nanoparticles have a mean particle diameter of 1 nm to 50 nm, and wherein said polymeric nanoparticles comprise, as polymerized units, at least one multiethylenically unsaturated monomer, and
(b) admixing said dispersion with at least one polymer.

15. An adhesive composition made by a process comprising:
(a) providing a concentrate of polymeric nanoparticles, wherein said concentrate has 80 weight% to 100 weight% polymeric nanoparticles based on the total weight of said concentrate, wherein said polymeric nanoparticles have a mean particle diameter of 1 nm to 50 nm, and wherein said polymeric nanoparticles comprise, as polymerized units, at least one multiethylenically unsaturated monomer, and
(b) admixing said concentrate with at least one polymer.

16. An adhesive composition made by a process comprising:
(a) providing a dispersion of droplets in an aqueous medium, wherein said droplets comprise monomer molecules and polymeric nanoparticles, wherein said polymeric nanoparticles have a mean particle diameter of 1 nm to 50 nm, and wherein said polymeric nanoparticles comprise, as polymerized units, at least one multiethylenically unsaturated monomer, and
(b) polymerizing said monomer molecules by an in-situ polymerization method.
